# EUROPEAN PATENT APPLICATION

(11) **EP 0 752 474 A1**
(43) Date of publication of application: **08.01.1997**
(21) Application number: 95110609.5
(22) Date of filing: 07.07.1995
(51) Int. Cl.: C12N 15/53, C12N 9/02, C12Q 1/68, C12P 19/00, C07K 14/505

(54) **Nucleic acid coding for CMP-N-acetyl-neuraminic acid hydroxylase and its use for the production of modified glycoproteins**

(71) Applicant: BOEHRINGER MANNHEIM GMBH, 68298 Mannheim (DE)
(72) Inventor: Schauer, Roland, D-24161 Altenholz-Klausdorf (DE); Schlenzka, Wiebke, D-24118 Kiel (DE); Kelm, Sörge, D-24146 Elmschenhagen (DE); Shaw, Lee, D-24103 Kiel (DE); Haselbeck, Anton, D-82362 Weilheim (DE); Honold, Konrad, D-82377 Penzberg (DE)

(57) **Abstract**

An isolated protein substantially free of contamination with cytochrome b₅ and cytochrome b₅ reductase, which
a) is a product of a prokaryotic or eukaryotic expression of an exogenous DNA, b) catalyzes the conversion of CMP-Neu5A to CMP-Neu5Gc, and c) is coded by the DNA sequence shown in SEQ ID NO:1; d) is coded by DNA sequences, which hybridize with SEQ ID NO:1 under stringent conditions; or e) is coded by DNA sequences, which, if there was no degeneracy of the genetic code, would hybridize under stringent conditions with the sequences defined in c) to d),
is useful for the production of CMP-N-acetylneuramic acid hydroxylase-deficient cells. Such cells are preferably used for the production of recombinant proteins lacking N-glycoloylneuraminic acid side chains.

## Description

### 1. Introduction

Sialic acids are a group of about 30 naturally occurring acidic sugars all of which are derived, in terms of structure, from N-acetyl-neuraminic acid (Neu5Ac) which is the most common sialic acid (R. Schauer (1985) *(1)*) and A. Varki (1992a) *(2)*). In most cases, they are located in the terminal position in the glycan chains of glycoconjugates. On account of this exposed position, they play an important role in cellular recognition, e.g. in the case of inflammatory reactions, maturation of immune cells, differentiation processes, hormone-, pathogen- and toxin binding (R. Schauer (1985) *(1)* and A. Varki (1992b) *(3)*). Also the biological effectiveness of glycoprotein hormones, such as, for instance, erythropoietin, is influenced by sialic acids (N. Imai et al. (1990) *(4)*).

N-glycoloylneuraminic acid (Neu5Gc) is distinguished from Neu5Ac by an additional hydroxyl group at the C5-N-acetyl residue. Neu5Gc is present in most animal groups having sialoglycoconjugates, the amount being specific with respect to type, tissue and development (A.P. Corfield and R. Schauer (1982) *(5)*; G. Reuter et al. (1988) *(6)* and D. Bouhours and J.-F. Bouhours (1983) *(7)*). Interestingly, Neu5Gc does not occur in healthy human and chicken tissues. Here Neu5Gc-containing glycoconjugates act as antigens and may induce the formation of antibodies. Historically, they are referred to as Hanganutziu-Deicher antigens and antibodies (H. Deicher (1926) *(8)*). Hanganutziu-Deicher antigens were detectable in a great number of human tumors (colon carcinoma, retinoblastoma, melanoma and carcinoma of the breast) as well as in chicken tumor tissues (H. Higashi et al. (1985) *(9)* and H. Higashi (1990) *(10)*). Although these amounts were very small (usually less than 1% of the total amount of sialic acid, often in the range of from 0.01 to 0.1%), they were capable of inducing the formation of Hanganutziu-Deicher antibodies (T. Higashihara (1991) *(11)*).

The biosynthesis of Neu5Gc occurs on the level of the CMP sialic acids through CMP-Neu5Ac hydroxylase (cytidine monophospho-N-acetylneuranoic acid hydroxylase). This enzyme catalyzes the conversion of CMP-Neu5Ac to CMP-Neu5Gc in the presence of molecular oxygen and NADH (L. Shaw and R. Schauer (1988) *(12*)). The electrons for the reductive activation of the oxygen are passed to the hydroxylase via an electron transport chain consisting of cytochrome b₅ reductase and cytochrome b₅ (Y. Kozutsumi et al. (1990) *(13)* and L. Shaw et al. (1994) *(14)*).

The formation of CMP-Neu5Gc takes place in cytoplasm. The proteins which take up CMP sialic acids in the Golgi apparatus (A. Lepers et al. (1989) *(15)*) and transfer them to resultant glycoconjugates (H.-H. Higa and J.C. Paulson (1985) *(16)*) accept both CMP sialic acids equally as a substrate. Hence the ratio of Neu5Ac and Neu5Gc in the glycoconjugates produced by the cells corresponds to the ratio of the respective CMP sialic acids in cytosol. The latter is dependent on the activity of the CMP-Neu5Ac hydroxylase.

The CMP-Neu5Ac hydroxylase has to date been isolated from mouse liver and pig submandibular glands to homogeneity and characterized (T. Kawano et al. (1994) *(18)*, P. Schneckenburger et al. (1994) *(19)* and W. Schlenzka et al. (1994) *(20)*). The purification of the enzyme was carried out using ion exchange chromatography, chromatography with immobilized triazin dyes, hydrophobic interaction chromatography and gel filtration (W. Schlenzka et al. (1994) *(20)*). The hydroxylase from mouse liver has been isolated in two different ways (P. Schneckenburger et al. (1994) *(19)*). From pig submandibular glands, due to the fact that the tissue is better accessible, larger amounts of enzyme can be isolated. From 500 g of tissue about 200 µg of hydroxylase protein can be obtained.

Both the CMP-Neu5Ac hydroxylase from pig submandibular glands and the one from mouse liver are soluble monomers having a molecular weight of 65 kDa. Their catalytic interactions with CMP-Neu5Ac and cytochrome b₅ are very similar to one another. The activity of both these enzymes seems to be dependent on an iron-containing prosthetic group.

Antibodies that are directed against this 65 kDa protein from pig submandibular glands inhibit the CMP-Neu5Ac hydroxylase activity and, in Western blotting, exhibit a strong cross-reaction with the purified enzyme from mouse liver

For the production of recombinant glycoproteins, mammalian cells are needed, because only in mammalian cells a complex glycosylation of proteins will take place. Non-human cell lines, especially hamster cells, exhibit considerable advantages with regard to the preparation of recombinant glycoconjugates (well characterized, high productivity). Therefore, human glycoproteins intended for therapeutic application often are prepared in CHO (Chinese hamster ovary) cells in which glycosylation of the proteins proceeds largely in the same way as glycosylation in human cells. A difference arises, however, in sialylation: Neu5Gc is inserted in a small amount (up to 3% of the total sialic acids) in correspondence with the normal glycosylation of these hamster cells (C.H. Hokke et al. (1990) *(17)*), whereas human glycoproteins do not contain any Neu5Gc, at least not in amounts detectable.

Proteins used for therapeutic application should, in particular in the case of long-term therapy, contain, on the corresponding glycoproteins and glycoconjugates respectively (proteoglycans, glycolipids), substantially no epitopes that do not occur in humans. The invention provides such recombinant proteins and methods for their production.

### Summary of the invention

One subject-matter of the invention is the molecular cloning of the CMP-Neu5Ac hydroxylase (also referred to hereinafter as "hydroxylase") and the isolation of nucleic acid molecules which encode said enzyme. Specifically preferred are nucleic acid molecules which hybridize to SEQ ID NO:1 under stringent conditions as set forth below. Nucleic acid molecules encoding the enzyme satisfy this criterion. In reconstitution experiments it has been shown that the recombinant hydroxylase is able to catalyze the conversion of CMP-Neu5A to CMP-Neu5Gc in vivo and in isolated cells and cell lines. Furthemore, the soluble recombinant hydroxylase is active in vitro.

A further subject-matter of the invention is a method of detecting nucleic acid molecules which code specifically for hydroxylase proteins. Preferably detected are DNA and mRNA species. Useful probes are nucleic acid molecules which bind specifically under the conditions of the test method applied, e.g. in situ hybridization, colony hybridization, Northern hybridization, or related techniques.

A further subject-matter of the invention is an oligonucleotide molecule which is complementary to a nucleic acid molecule of hydroxylase occurring in mammalian cells. Such oligonucleotide molecules which have, in general, about 15 to 50 bases for being specific, are useful for inhibiting expression of hydroxylase, preferably on the DNA or mRNA level. Such oligonucleotide molecules are useful as antisense agents in the treatment of tumor therapy, especially in the treatment and prevention of metastases.

A further subject-matter of the invention is a nucleic acid molecule, which inhibits the expression of hydroxylase in a host cell by altering the hydroxylase gene, preferably by homologous recombination, in such a way that proteins with hydroxylase activity are not expressed in said host cell in an amount which is sufficient for a detectable Neu5Gc glycosylation of recombinant proteins produced in said host cell.

A further subject-matter of the invention is a method for the production of recombinant glycoproteins in mammalian cells wherein a mammalian cell is transfected with a nucleic acid molecule, which secures expression of said desired glycoprotein, culturing the cells, recovering the protein from the cell or the cell supernatant, characterized in that as a mammalian cell there is used a mammalian cell with no detectable Neu5Gc glycosylation (hydroxylase deficient), preferably in a cell in which the gene encoding CMP-Neu5Ac hydroxylase is inactivated, as well as such cells and so produced recombinant glycoproteins.

### Detailed description of the invention

The invention comprises a recombinant protein having hydroxylase activity. The protein can be defined by its DNA sequence and by the amino acid sequence derived therefrom (see, e.g., SEQ ID NO:2). The hydroxylase protein can occur in natural allelic variations which differ from individual to individual. Such variations of the amino acids are usually amino acid substitutions. However, they may also be deletions, insertions or additions of amino acids to the total sequence. The hydroxylase protein according to the invention - depending, both in respect of the extent and type, on the cell and cell type in which it is expressed - can be in glycosylated or non-glycosylated form.

The term "hybridize" denotes a binding of such a sequence to SEQ ID NO:1 under standard stringent hybridization conditions as set forth below.

The hydroxylase can now be produced by recombinant means. Non-glycosylated hydroxylase is obtained when it is produced recombinantly in prokaryotes. With the aid of the nucleic acid sequences provided by the invention it is possible to search for the hydroxylase gene or its variants in genomes of any mammalian cells and tissue, preferably in such cells or tissue which are positive for the hydroxylase (e.g. liver cells and submandibular glands), to identify these and to isolate the desired gene coding for the hydroxylase. Such processes and suitable standard stringent hybridization conditions are known to a person skilled in the art and are described for example by J. Sambrook (1989) *(22)* and B.D. Hames, S.G. Higgins (1985) *(23)*. In this case the standard protocols described in these publications are usually used for the experiments. Particular reference is made to section IX, "Hybridization of radiolabeled probes to immobilized nucleic acid", page 947-962 with regard to the hybridization of nucleic acid molecules, and to section XI, page 1145-1161, "Condition for hybridization of oligonucleotide probes" with regard to the hybridization of oligonucleotide probes, both quotations beings incorporated herein by reference. Standard stringent conditions are also described, for example, by Höltke and Kessler (1990) *(24)*.

More specifically, stringent conditions as used herein refer to hybridization in 1 mol/l NaCl, 1% SIDS and 10% dextran sulfate. This is followed by two washes of the filter at room temperature of 5 minutes in 2 x SSC and one final wash for 30 minutes. This final wash may be at 0.5 x SSC, 0.1% SDS, more preferably at 0.2 SSC, 0.1% SDS, and most preferably at 0.1 x SSC, 0.1% SDS, final wash taking place at 65°C. Those of ordinary skill in the art will recognize that other conditions will afford the same degrees of stringency and are encompassed by the phraseology "under stringent conditions" and are encompassed herein.

Cells and tissue which are positive for the hydroxylase can be identified by an antibody which is specific for the hydroxylase. Such specific antibodies can be easily produced according to Schlenzka et al. *(20)* which is incorporated herein by reference.

The use of recombinant DNA technology enables the production of numerous hydroxylase derivatives. Such derivatives can for example be modified in individual or several amino acids by substitution, deletion or addition. The derivatization can for example be carried out by means of site directed mutagenesis. Such variations can be easily carried out by a person skilled in the art (J. Sambrook *(22)*, B.D. Hames *(23)*). It merely has to be ensured that the characteristic properties of the hydroxylase activity (polysialyl transferase activity) are preserved.

The invention therefore comprises an isolated hydroxylase which
a) is a product of a prokaryotic or eukaryotic expression of an exogenous DNA,
b) catalyzes the conversion of CMP-Neu5A to CMP-Neu5Gc, and
c) is coded by the DNA sequence shown in SEQ ID NO:1,
d) is coded by DNA sequences which hybridize under stringent conditions with SEQ ID NO:1, or
e) is coded by DNA sequences which if there was no degeneracy of the genetic code, would hybridize under stringent conditions with the sequences defined in c) to d).

A protein is preferred which is coded by nucleotides 42 to 1307 of SEQ ID NO:1 or by DNA sequences which due to genetic code degeneracy would code for a polypeptide with the same amino acid sequence.

The invention further concerns a nucleic acid molecule, which secures the expression in a prokaryotic or eukaryotic host cell of a protein with hydroxylase activity and is selected from the group comprising
a) the DNA sequence shown in SEQ ID NO:1 or the complementary sequences,
b) nucleic acid sequences which hybridize under stringent conditions with SEQ ID NO:1, or
c) nucleic acid sequences which, if there was no degeneracy of the genetic code, would hybridize under stringent conditions with one of the sequences stated in a) or b).

Preferably, the nucleic acid molecule codes for a protein which catalyzes the conversion of CMP-Neu5A to CMP-Neu5Gc.

With the aid of these nucleic acid molecules the protein according to the invention can be obtained in a reproducible manner and in large amounts. For this the nucleic acid molecule is integrated into suitable expression vectors, such as an exogenous nucleic acid molecule, according to methods familiar to a person skilled in the art for expression in prokaryotic or eukaryotic organisms, such as prokaryotic host cells or eukclryotic host cells. Such an expression vector preferably contains a regulatable/inducible promoter to which the coding sequence is operably linked. These recombinant vectors are then introduced for the expression into suitable host cells such as e.g. E. coli as a prokaryotic host cell or Saccharomyces cerevisiae, Terato carcinoma cell line PA-1 sc 9117 (Büttner et al. (1991) *(25)*), insect cells, such as Sf9, and all insect cells transfected with baculovirus vector, CHO or COS cells as eukaryotic host cells and the transformed or transduced host cells are cultured under conditions which allow an expression of the heterologous gene. The isolation of the protein can be carried out according to known methods from the host cell or from the culture supernatant of the host cell. Such methods are described for example by Ausubel I. and Frederick M. (1992) *(26)*. Also in vitro reactivation of the protein may be necessary, if, for example, after expression in prokaryotes, an insoluble and inactive protein is obtained.

A nucleic acid molecule coding for a protein with hydroxylase activity and/or consisting of the nucleotides 42 to 1307 of SEQ ID NO:1 is preferably used for the recombinant production of the protein according to the invention.

It is possible to provide a test on the basis of the nucleic acid sequences of the hydroxylase provided by the invention which can be used to detect nucleic acid molecules which code specifically for hydroxylase. Such a test can for example be carried out in cells or cell lysates. Such a test can be carried out by means of nucleic acid diagnostics. In this case the sample to be examined is brought into contact with a probe which would hybridize with the nucleic acid molecules coding for the hydroxylase with the sequences which are specific for hydroxlyse (nucleotides 42 to 1307 of SEQ ID NO:1). A hybridization between the probe and nucleic acid molecules from the sample indicates the presence of expressed hydroxylase. Such methods are known to a person skilled in the art and are for example described in WO 89/06698 *(27)*, EP-A 0 200 362 *(28)*, USP 2,915,082 *(29*), EP-A 0 063 879 *(30)*, EP-A 0 173 251 *(31)*, EP-A 0 128 018 *(32)*. In a preferred embodiment of the invention, the nucleic acid molecule of the sample which codes for a hydroxylase is amplified before testing, e.g. by the well-known PCR technique. A derivatized (labelled) nucleic acid probe is usually used in the field of nucleic acid diagnostics. This probe is brought into contact with a carrier-bound denatured DNA or RNA from the sample and in this process the temperature, ionic strength, pH value and other buffer conditions are selected in such a way that - depending on the length of the nucleic acid molecule sample and the resulting melting temperature of the expected hybrid - the labelled DNA or RNA can bind to homologous DNA or RNA (hybridization, see also J. Mol. Biol. 98 (1975), 503 *(33)*; Proc. Natl. Acad. Sci. USA 76 (1979), 3683 *(34)*). Suitable carriers are membranes or carrier materials based on nitrocellulose (e.g. Schleicher and Schüll, BA 85, Amersham Hybond, C.) reinforced or bound nitrocellulose in a powder form or nylon membranes derivatized with various functional groups (e.g. nitro group) (e.g. Schleicher and Schüll, Nytran; NEN, Gene Screen; Amersham Hybond M.; Pall Biodyne).

The hybridized DNA or RNA is then detected by incubating the carrier, after thorough washing and saturation to prevent unspecific binding, with an antibody or antibody fragment. The antibody or antibody fragment is directed towards the substance incorporated into the nucleic acid probe during the derivatization. The antibody is in turn labelled. It is, however, also possible to use a directly labelled DNA. After incubation with the antibodies, it is washed again in order to only detect specifically bound antibody conjugates. The determination is then carried out via the label of the antibody or antibody fragment according to well-known methods.

The detection of the hydroxylase expression can be carried out for example as:
- in situ hybridization with immobilized whole cells using immobilized tissue smears and isolated metaphase chromosomes,
- colony hybridization (cells) and plaque hybridization (phages and viruses),
- Northern hybridization (RNA detection),
- serum analysis (e.g. cell type analysis of cells in serum by slot-blot analysis),
- after amplification (e.g. PCR technique).

Since hydroxylase preferably is regulated on the mRNA level, it is preferred to carry out, for detecting the hydroxylase expression, a hybridization with the mRNA of the cell to be examined.

The invention therefore includes a specific method for the detection of nucleic acid molecules which code for a hydroxylase protein which is characterized in that the sample to be examined is incubated with a nucleic acid probe which is selected from the group comprising nucleic acid molecules or specific oligonucleotides which hybridize with the nucleotides 42 to 1307 of SEQ ID NO:1, the nucleic acid probe is incubated with the nucleic acid molecules from the sample and the hybridization of the nucleic acid molecules in the sample and nucleic acid probe is detected, if desired, via a further binding partner.

As the abnormal activation of an otherwise dormant hydroxylase gene is found in mammalian tumors, such an assay is a valuable test in tumor diagnosis.

Another feature of the invention are oligonucleotide molecules which specifically inhibit the expression of hydroxylase in mammalian cells. It has been found that an oligonucleotide molecule of about more than 15 to 17 nucleotides in length must have a unique sequence relative to the entire human genome and is therefore specific. Further, it was shown that short antisense oligonucleotides can be imported into cells and exhibit inhibition (Zamecnik et al. (1986) *(58)*). In particular, oligonucleotide molecules having 15 to 50, preferably 15 to 25, bases are suitable for use.

A further embodiment of the invention is an oligonucleotide molecule which hybridizes in a manner which is specific for nucleic acid molecules of mammalian hydroxylase with a part or all of SEQ ID NO:1.

Therefore, such an oligonucleotide molecule which is complementary to, and designed on the basis of, the above-mentioned sequences of mammalian hydroxylase genes is useful for inhibiting the expression of hydroxylase in vivo and in isolated mammalian cells.

The term "hybridizes in a manner which is specific for a nucleic acid molecule of mammalian hydroxylase" means that such a nucleic acid molecule or oligonucleotide molecule in mammalian cells, , especially in human cells, binds to the nucleic acid molecules which code for a hydroxylase in said cells. A specific binding will be present if these nucleic acid molecules inhibit the expression of hydroxylase in a considerable manner (more than 50%, preferably more than 80%, or more than 90%) and in such fashion that the other metabolism processes of the cell are not impaired.

An oligonucleotide molecule or a nucleic acid molecule which is to hybridize specifically with the hydroxylase nucleic acid molecule will at least in its essential part be complementary to sequences in the coding region of nucleotides 42 to 1307 of SEQ ID NO:1. Standard hybridization conditions are described in Sambrook et al. (1989) *(22)* as already stated.

A preferred oligonucleotide molecule interferes in a sequence-specific manner with processes such as the translation of hydroxylase mRNA into the protein by binding to hydroxylase nRNA. Further oligonucleotide molecules which are suitable for use are oligonucleotide molecules which are complementary to genomic DNA which can interact in forming a triple helical structure (M. Cooney et al. (1988) *(35)* and Duvall-Valentine et al. (1992) *(36)*).

As the antisense oligonucleotide molecules, preferably oligonucleotide derivatives, such as phosphotriester, methylphosphonates, phosphorothioates or substituted oligonucleotides, such as acridine, interchalating coupled oligonucleotides or α-anomers (J.J. Toulmé and C. Hélène (1988) *(37)*) are used. Phosphorothioates and methylphosphonates are specifically preferred. Such oligo derivatives can be synthesized according to the state of the art, for example by automated technology (S. Beaucage and M. Caruthers (1981) *(38)*; G. Zon and T. Geiser (1991) *(39)*; C.A. Stein et al. (1991) *(40)*; P. Miller (1991) *(41)*). Especially phosphothioates and methylphosphonates are oligos which are useful, because they are resistant against serum and intracellular nucleases. Further useful antisense oligonucleotides which are nuclease-resistant are described in P.S. Miller et al. (1985) *(42)*.

It is, in principle, possible to use naked antisense oligonucleotide molecules according to the invention, because these oligonucleotide can be taken up by cells non-specifically. However, such oligonucleotides are integrated in the cells at a low efficiency. Therefore, in general, a delivery system for antisense oligos or a targeting vector is preferred especially for in vivo use.

The transfection of mammalian cells in vitro, especially of hamster cells, is accomplished according to the methods known in the state of the art, such methods being, for example, electroporation or microinjection. After transfection, the cells which are defective in hydroxylase activity are separated, for example, by single cell separation with subsequent determination of hydroxylase activity in these cells, for example, by Western blotting.

By this method, the invention provides mutants of mammalian cells normally (in their initial, unmodified stage) having hydroxylase activity, said mutants being defective in the hydroxylase activity. Cells, which normally have hydroxylase activity, mostly are non-human cells such as hamster, mouse, rat, pig cells, and hybridomas derived therefrom.

The term "defective in the hydroxylase activity" means that the hydroxylase activity is reduced by about 80%, preferably more than 90%, as compared with the non-mutated initial cell. The determination of hydroxylase activity is accomplished, suitably, by means of an enzymatic assay as described in L. Shaw et al. (1994) *(14)*. Such hydroxylase-deficient mammalian cells are produced by transfecting said cells with a nucleic acid molecule, which hybridizes under stringent conditions in a manner which is specific for a nucleic acid molecule with SEQ ID NO:1 or related RNA species, and isolating from the cell mixture at least one cell, which is deficient in said hydroxylase activity.

Such cells can be used preferably for the recombinant production of glycoproteins in non-human cell lines, whereby the cell-produce glycoprotein does not contain N-glycolylneuraminic acid. The method according to the invention can be used for the production of glycoproteins, such as erythropoietin (EPO), colony-stimulating factors (GCSF, GMCSF, MegCSF or thrombopoietin). The invention further comprises recombinant glycoproteins without attached N-glycolylneuraminic acid, substantially free, preferably completely free, of other human proteins.

The cells, which are deficient for hydroxylase activity, are produced preferably by homologous recombination. Such methods are described, for example, in US Patent No. 5,272,071 *(75)*, M.R. Capecchi (1989) *(76)*, G.Y. Wu et al. (1991) *(77)* and WO 91/13151 *(78)*. For homologous recombination it is preferred to use a targeting vector containing as much hydroxylase homology as possible for having a high targeting frequency. The targeting vector is such that, after homologous recombination, it will modify the hydroxylase gene in such a way that no longer any protein, or merely negligible amounts of protein, will be formed in the targeted cell so that the targeted cell will be deficient in hydroxylase activity.

It is especially preferred to use a vector in which this hydroxylase sequence or part thereof is interrupted by a selectable gene, such as NeoHPRT. As cells there are particularly preferred hamster cells, such as CHO cells, especially such cells which are deficient in the dihydrofolate reductase gene (DHFR gene). Such DHFR-deficient cells are described, for example, in US Patent Nos. 4,399,216 *(79)* and 4,634,665 *(80)*.

The targeting vector preferably contains about 5 to 10 kB of the genomic DNA sequence. This sequence may comprise introns and exons of the gene and may contain, in addition, 5'- or 3'-non-coding sequences. Preferably, genomic DNA from the cells to be mutated (e.g. CHO) is used. With a corresponding homology, there may also be used, however, genomic DNA from cells of another species. Cloning of the genomic DNA can be accomplished by screening of λ-DNA libraries using DNA samples, which are derived from SEQ ID NO:1.

Further, the targeting vector preferably contains a selectable artificial marker. To accomplish a positive selection, for instance, neomycinphosphotransferase (neo) which makes possible G-418 selection can be applied. With the use of this marker it is possible to isolate cells containing the targeting vector DNA. The marker may also be applied as a mutagen if, for instance, it is cloned into a coding exon or replaces a coding exon.

To accomplish a negative selection, for instance, the Herpes simplex thymidine kinase (HSV-TK) which effects an Acyclovir or Ganciclovir sensitivity can be used. This negative marker can be incorporated in the targeting vector in such a way that, in the case of a homologous recombination, it will not be integrated in the DNA of the target cells.

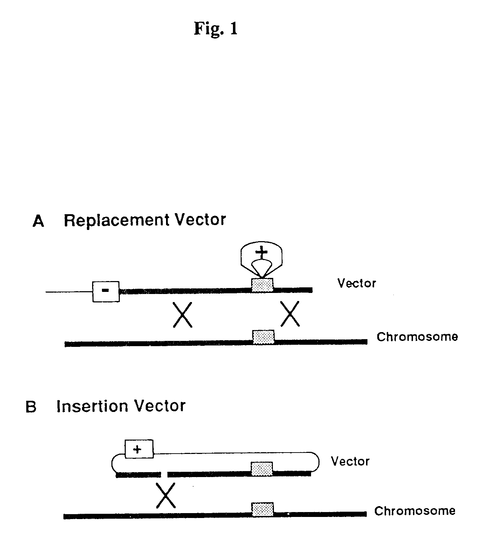

Two types of targeting vectors are applied: Replacement vectors or insertion vectors. They are distinct in their structure and, after homologous recombination, result in different integration products (A.L. Joyner (1993) *(81)*). These two vectors are represented schematically in Figure 1.

As methods for the targeting into the hydrolase gene of cells, microinjection or electroporation are preferred. Targeted clones are detected either by selection on the marker gene introduced or by PCR assay. For this PCR assay, two primers are used: Primer 1 is specific to the target locus and is preferably about 400 bp upstream of the 5'-end of the targeting fragment in the genome of the cell, and primer 2 is specific to the incoming DNA. Specific PCR amplification can occur only if a targeting event juxtaposes these two primers. The targeted clones were isolated from the PCR-positive pools which contain about 200 independent clones, which show the marker activity. Cells from this pool are diluted into 96 smaller pools with about 10 cells in each. Alter expansion, these smaller pools were rescreened by PCR for the presence of the targeting event. Positive signals now corresponded to mixtures having one targeted cell for every 10 non-targeted cells. One of the enriched pools was then diluted into microtiter dishes so that only 10 to 20% of the wells received a cell. After growth to adequate density, samples were removed and analyzed again by PCR assay. By this way, clones of the positively targeted cells can be isolated.

For further analyzing, one isolate of the targeted clone was expanded and used to prepare genomic DNA for Southern blot analysis.

A further subject-matter of the invention is a soluble complex for targeting an oligonucleotide molecule according to the invention which hybridizes in a manner which is specific for nucleic acid molecules of the hydroxylase with a part or all of SEQ ID NO:1, complexed with an oligonucleotide molecule binding agent, which complexes the oligonucleotide molecule under extracellular conditions and releases said oligonucleotide molecule under intracellular conditions as an oligonucleotide molecule specifically bindable to polysialyl transferase nucleic acid molecule.

Such delivery systems are well-known in the state of the art. For example, oligonucleotides are covalently coupled to polycations, such as polylysine (M. Lemaitre et al. (1987) *(43)*). Further delivery systems using polycation conjugates (e.g. transferrin) are described in WO 92/20316 *(44)*, USP 5,166,320 *(45)*, WO 92/19281 *(46)*, WO 92/13570 *(47)*, EP-A 0 388 758 *(48)*, WO 93/07283 *(49)*, WO 92/17210 *(50)*, WO 91/17773 *(51)*, WO 93/04701 *(52)* and transfer peptides as specified in PCT/EP94/01147 *(53)* are also suitable for use.

The efficacy of the internalization of the nucleic acid molecules according to the invention in cells can be improved by binding the nucleic acid molecule to amphiphilic molecules, such as polyethylene glycol in a complex. Further preferred are the use of transfection reagents, such as DOTMA (WO 91/06309 *(54)* and WO 91/17424 *(55)*). Liposomes and dendromers are also useful.

In order to attain cell specificity, the nucleic acid molecules according to the invention can be coupled non-covalently to conjugates from a DNA-binding substance (e.g. polycations) and/or a cell-specific ligand (e.g. protein, preferably PSA specific antibody) (Wu and Wu (1987) *(56)*; Wu et al. (1988) *(57)*). Further, internalization of the nucleic acid molecules in cells is accomplished by means of a soluble DNA carrier system consisting of a chemically synthesized conjugate comprising mannose and lactose as the ligands (P. Midoux et al. (1993) *(58)*). EP-A 0 388 758 *(48)* discloses chemically synthesized transferrin polycation conjugates which form complexes with polyanionic nucleic acid molecules. By means of the binding to the transferrin receptor these complexes can be internalized in the target cells.

It is also known to use conjugates of polylysine and asialoglycoprotein (Wu et al. (1988) *(57)*) or with a galactose ligand (Plank et al. (1992) *(59)*) in the complexes. As ligands there were also employed inactivated adenoviruses (Cotten et al. (1992) *(60)*; Wagner et al. (1992) *(61)*) or haemagglutinin infusion peptides (Wagner et al. (1992) *(62)*). WO 93/07283 *(49)* also describes, with regard to non-viral gene transfer, a "2-ligand-system" comprising a DNA-binding (polycationic) portion (substance with affinity towards nucleic acid molecules) and an internalization factor for the take-up of DNA in the cell. To release the complexes from the endosomes into cytoplasm, there may be added to these complexes, as described in WO 93/07283 *(49)*, a socalled endosomolytic agent which corresponds, for example, to a virus or a virus component (e.g. adenovirus or influenza haemagglutinin).

As antisense oligos there are preferably selected oligos which hybridize with approximately a part or all of nucleotides 42 to 1307 of SEQ ID NO:1.

If the carrier for the oligonucleotide molecule is a conjugate from a cell-specific ligand and a DNA binding substance, these substances are preferred to be covalently linked and the linkage typically is a peptide bond. This can be formed, for example, with a water-soluble carbodiimide as described by G. Jung et al. (1981) *(63)*. An alternative linkage is a disulfide bond.

The cell-specific agent can be a natural or synthetic ligand (for example, a protein polypeptide, glycoprotein, etc.) or it can be an antibody or an analogue thereof which specifically binds to a cellular surface structure which then mediates internalization of the bound complex. Such antibodies are, for example, anti-CD3 or anti-CD4 antibodies. After binding, these antibodies are internalized.

Typically, the cell-specific binding agent is a ligand which binds to a cell-surface receptor. Preferably there are employed receptors which are specific for such tissue cells from which the tumor to be treated originates.

It is specifically preferred to use the antisense oligos for the treatment of tumor diseases involving a high metastasis potential (small cell lung carcinoma, medulloblastoma, Wilms' tumor and lymphoid tumor W.F. Kern et al. (1993) *(65)*). Tumor antigens that are suitable for use as surface receptors are, for instance, the tumor antigens.

It is further preferred that the employed cell-specific ligand acts as a substance which facilitates the internalization of the oligonucleotide molecules according to the invention. Such internalization factors are, for example, transferrin or anti-CD4-antibody.

The optional ratio of the cell-specific binding agent to the oligonucleotide molecule and the oligonucleotide binding agent in the complexes can be determined empirically. When polycations are used, the molar ratio of the components will vary depending on the size of the polycation and the size of the oligonucleotide. To form the complex, the oligonucleotide molecules and carriers are mixed and incubated under conditions conductive to complexation. For example, the oligonucleotide molecule and carrier can be mixed at the appropriate ratio in 2 mol/l NaCl and the solution can be diluted to 0.15 mol/l and filtered to provide an administerable composition.

The oligonucleotide molecules or the molecular complexes of this invention can be administered parenterally. Preferably, it is injected intravenously. The complex is administered in solution in a physiologically acceptable vehicle.

Vector pWSPH.01 which contains the nucleotides 150 to 1986 of SEQ ID NO:1 was deposited under the Budapest Treaty with Deutsche Sammlung von Mikroorganismen und Zellkulturen GmbH (DSM), Mascheroder Weg 1b, D-38124 Braunschweig on 24 May 1995 and was accorded Accession Number DSM 10009.

A second vector pWSPH.03 which contains the nucleotides 1 to 211 of SEQ ID NO:1 was deposited under the Budapest Treaty with Deutsche Sammlung von Mikroorganismen und Zellkulturen GmbH (DSM), Mascheroder Weg 1b, D-38124 Braunschweig on 24 May 1995 and was accorded Accession Number DSM 10010.

Due to the fact that the occurrence of minor errors in the sequencing of DNA sequences cannot be ruled out, it is pointed out that in the event of any discrepancy between the sequence contained in the vectors pWSPH.01 and pWSPH.03 and the sequence stated in SEQ ID NO:1, the sequence accessible, to the person skilled in the art, from said vectors should be resorted to.

The following examples and sequences as well as the figure are provided to aid the understanding of the present invention, the true scope of which is set forth in the appended claims. It is understood that modifications can be made in the procedures set forth without departing from the spirit of the invention.
- **Figure 1:**: Vectors useful for the production of hydroxylase-deficient host cells.
The fat line shows the vector homology to the target locus (hydroxylase gene). The thin line shows the bacterial plasmid sequence (e.g. pUC). The dotted rectangle represents a hydroxylase exon. The positive selection marker is represented by the symbol "+".
A) Replacement Vector: The positive selection marker interrupts the target homology region of the hydroxylase gene, preferably in an upstream exon, in order to attain a zero mutant. The negative selection marker is represented by the symbol "-". The replacement vector is linearized prior to transfection outside of the target homology region.
B) Insertion Vector: A positively selectable marker can be cloned into the hydroxylase-homologous sequence or into the vector background. A double strand break within the homologous targeting sequence is inserted prior to transfection.
Targeting vectors can be inserted in the production cell lines by means of electroporation.
By means of DNA (Southern blot, PCR), RNA (Northern blot) and protein analysis it can be verified whether the hydroxylase gene has been mutated successfully. In the event that an intact allele remains in the cells, another knock out preparation can be carried out, using alternative selection markers.

### Sequence Listing:

- SEQ ID NO:1: denotes nucleotide and deduced protein sequence of the hydroxylase cDNA.
- SEQ ID NO:2: denotes protein sequence deduced from cDNA.

### Example 1

### Purification of CMP-Neu5Ac hydroxylase

All procedures were performed at 4°C. Pig submandibular glands were removed from freshly slaughtered animals and stored frozen at -20°C. Thawed tissue was minced and homogenized in 50 mM HEPES/NaOH (pH 7.4) (5 ml/g wet mass of tissue) using an Ultraturrax. The resulting homogenate was centrifuged at 10,000 *g* for 30 min and the pellet was discarded. The mucin and remaining particulate material in the 10,000 *g* supernatant were precipitated by the addition of 2% (by mass) N-cetyl-N,N,N-trimethylammonium bromide dissolved in 50 mM HEPES/NaOH (pH 7.4), (final concentration 0.2%), followed by centrifugation at 100,000 *g* for 1 h. CMP-Neu5Ac hydroxylase was purified from this high-speed supernatant. The mucin content was estimated by quantification of sialic acids using the orcinol reagent (R. Schauer et al. (1978) *(82)*.

The mucin-free high-speed supernatant (1.1 l) was fractionated in a batch procedure using 300 ml Q-Sepharose slurry equilibrated with 10 mM HEPES/NaOH (pH 7.4). For each step, the Q-Sepharose was sedimented by centrifugation at 75 *g* for 5 min. Non-bound protein was removed from the Sepharose with 1.75 l 10 mM HEPES/NaOH (pH 7.4) and bound protein including hydroxylase activity was eluted with 500 ml 10 mM HEPES/NaOH (pH 7.4) containing 400 mM NaCl.

After a 4-fold dilution with 50 mM HEPES/NaOH (pH 7.4), the Q-Sepharose eluate was applied to a Cibacron Blue 3GA-Agarose column (5 x 11 cm) equilibrated with 50 mM HEPES/NaOH (pH 7.4) containing 100 mM NaCl, the flow rate being about 2 ml/min. Non-bound protein was removed with the equilibration buffer and the column was developed with a 1.2 l linear gradient from 0.1 to 1.5 M NaCl in 50 mM HEPES/NaOH (pH 7.4). The fractions with the greatest hydroxylase activity were pooled, concentrated and, after desalting, applied to a column of Reactive Brown 10-Agarose (1.5 x 5.5 cm) equilibrated with 50 mM HEPES/NaOH (pH 7.4) at a flow rate of 0.1 ml/min. After washing with equilibration buffer, elution was performed with a 120 ml linear gradient from 0 to 2 M NaCl in equilibration buffer.

After pooling and concentration of the most active fractions, ammonium sulphate was added to 41% saturation and the sample applied to a column of Hexyl-Agarose (1.5 x 6.0 cm) equilibrated in 50 mM HEPES/NaOH (pH 7.4) containing ammonium sulphate at 41% saturation. Washing of the column with the equilibration buffer was followed by elution of bound protein with a 140 ml linear gradient of (NH₄)₂SO₄ from 41 to 0% saturation in 50 mM HEPES/NaOH (pH 7.4), the flow rate being 1 ml/min.

The final purification on Superose S.12 was performed using the desalted Hexyl-Agarose pool after concentration to a volume of 200 µl. The Superose S.12 column was equilibrated in 50 mM HEPES/NaOH (pH 7.4) containing 100 mM NaCl and calibrated with cytochrome c (12.5 kDa), carbonic anhydrase (29 kDa), ovalbumin (45 kDa) and bovine serum albumin (66 kDa). The flow rate was 0.2 ml/min and 200 µl fractions were collected.

The protein concentrations were measured by the method of Bradford (1976), using Bio-Rad reagent (Bio-Rad, Munich, FRG). Protein samples were concentrated by ultrafiltration on membranes with a *M*_{*r*} cutoff of 10 or 20 kDa (Sartorius, Göttingen, FRG).

Samples of each purification step were analyzed by SDS-PAGE on a gel consisting of a 4% stacking gel and a 13% resolving gel.

### Example 2

### Cloning the CMP-Neu5AC hydroxylase

With the use of three of the amino acid sequences (peptides 1, 2 and 5), degenerated oligonucleotide primers for both orientations, taking into account the codon usage in pigs, were prepared mRNA from pig submandibular glands was transcribed into single-strand cDNA. By means of this cDNA and the primers, PCR (polymerase chain reaction) at various conditions was carried out. In doing so, all possible primer combinations were investigated in the first reaction series. The products resulting from these preparations were applied in a second series of PCR reactions in which the reverse primers were exchanged (nested primers). In doing so, two PCR products (96 bp and 411 bp) were obtained which suggest the following order of the peptides: 1, 2 and 5.

The PCR product having a size of 96 bp was transformed after ligation into a pUC vector by means of electroporation in E.coli cells, strain XL1 blue. The plasmid insert of a positive clone was sequenced. The protein sequence derived therefrom contains the regions of the peptide pieces 2 and 5 that were used to prepare the primers, as well as the adjacent regions. Thus this PCR product is a part of the hydroxylase gene.

From mRNA of pig submandibular glands, there was prepared an oligo (dt)-primed cDNA library in λ-phagen ZAP II (Stratagene). The 96 bp PCR product, after being labelled with digoxigenin, was employed as a probe to search within this cDNA library from pig submandibular glands. In doing so, several positive clones were found; the longest of these clones, which had a length of about 1800 bp, was characterized and sequenced. In the nucleotide sequence, a reading frame of about 1270 bp, which was open at the 5' end, was found. The peptide sequence derived therefrom contains all known peptide pieces of the CMP-Neu5Ac hydroxylase. This confirms that the clone found codes for about 80% of the hydroxylase protein.

### Example 3

### Recombinant expression of fusion-free hydroxylase in E. coli

The DNA sequence coding for hydroxylase is modified in such fashion as to allow for efficient expression in E. coli.

For expression, an expression plasmid is transfected into a suitable E. coli strain. Such strains are, in the case of the use of an expression plasmid under the control of lac repressor such as the expression plasmid p11379, strains which possess a sufficiently high intracellular concentration of lac repressor. These kinds of strains can be prepared by transfection of a second plasmid such as pREP4 (Diagen GmbH), pUBS 500 or pUBS520 (Brinckmann et al. (1989) *(67)*). The applied E. coli strains should preferably have a low protease activity of the cells proper, as is the case, for instance, with E. coli UT5600 (Earhart et al. (1979) *(67)*), E. coli BL21 (Grodberg and Dunn (1988) *(68)*) or E. coli B. Then, expression cultivation is accomplished in a fashion according to the state of the art. For recovery, hydroxylase obtained as a protein aggregate from E. coli is processed according to the procedures described in EP 0 241 022 *(69)*, EP 0 364 926 *(70)*, EP 0 219 874 *(71)* and DE-A 40 37 196 *(72)*.

In detail, for example the following procedure is applied for this purpose: hydroxylase-containing protein aggregates from E.coli fermentations (socalled "inclusion bodies") are solubilized in 6 M guanidinium hydrochloride, 100 mM TrisHCl at pH 8, 1 mM EDTA, subsequently adjusted to a pH of 3 to 4 and dialyzed against 4 M guanidinium hydrochloride at pH 3.5. The renaturing of the solubilized protein is then carried out in 1 M arginine at pH 8, 1 mM EDTA, 5 mM GSH (glutathione, reduced) and 0.5 mM GSSG (glutathione, oxidized). From the renaturing preparation, hydroxylase can be obtained, for instance, after addition of 1.4 M ammonium sulfate by adsorption to hydrophobic gel matrices such as Fractogel TSK Butyl (E. Merck, Darmstadt) and subsequent elution in 20 mM TrisHCl at pH 7.

### Example 4

### Recombinant expression of hydroxylase in mammalian cells

For this, the cDNA is ligated into a vector in which it is transcribed into mammalian cells, on the basis of a strong promoter-enhancer system. Such promoters and enhancers are mostly from viruses such as SV40, hCMV, polyoma or retroviruses. As an alternative there can also be applied promoter-enhancer systems which are specific to a certain cell type or tissue type, such as, for instance, WAP-, MMTV- or immune globuline promoter, or systems which are inducible, such as, for instance, metallothioneine promoter. This kind of vector supplements the hydroxylase cDNA (if the latter is used) with donor and acceptor signals for RNA processing as well as a signal for poly-A-addition. For example, pCMX-pL1 (Umesono et al. (1991) *(73)*) is such a suitable vector. Into the one and only EcoRI cleavage site of this vector the hydroxylase cDNA provided with EcoRI linkers is ligated, wherein it is ensured by restriction analysis with the aid of the other cleavage sites in the polylinker of this vector that the hydroxylase cDNA is oriented in reading direction of the CMV promoter. An absolutely analogous procedure is applied when cloning into other vectors, e.g. into pCDNA3 (Invitrogen, San Diego/USA) or pSG5 (Stratagene, LaJolla/USA). The DNA of the so obtained expression plasmids is prepared from E. coli and transfected into the mammalian cells, applying techniques that are specific to the cell types in the particular case (Methods of Enzymology 185 (Gene Expression Technology), ed. David V. Goeddel, Academic Press 1991, section *(74)*). After transfection, the cells are cultured in MEM (Gibco) without addition of fetal calf serum, whereby hydroxylase is detectible in the cell culture supernatant after 48 hours.

### Example 5

### Digoxigenin(DIG)-labelled RNA probes for diagnostic purposes

For preparing a probe, the fragment to be employed for hybridization was cloned in a suitable transcription vector (wit a T3, T7 or SP6 promoter). For labelling, 1 to 2 µg of the plasmid were linearized, subsequently purified by means of phenol/chloroform extraction and precipitated with ethanol.

Reaction batch:
1 to 2 µg DNA (dissolved in DEPC-treated H₂O), 2 µl 10 x transcription buffer (Boehringer Mannheim), 40 U RNA polymerase (T3, T7 or SP6), 2 µl NTP/DIG-UTP mixture (10 mM ATP, CTP, GTP; 6.5 mM UTP; 3.5 mM DIG-UTP) and 1 µl RNase inhibitor (20 U/µl) were filled with H₂O to a volume of 20 µl.

The preparation was incubated for two hours at 37°C. Subsequently the DNA was removed by the addition of 2 µl RNase-free DNase I (10 U/µl) and by further incubation for 15 minutes at 37°C. The reactions were stopped by adding 1 µl 0.5 M EDTA (pH 8.0), and the synthesized RNA was precipitated with 0.1 vol. 3 M sodium acetate (pH 5.2) and 2.5 vol. ethanol. The RNA was washed once with 70% ethanol, dried and then dissolved in 100 µl H₂O (DEPC-treated). The labelling efficiency was determined in that dilution series of the labelled sample and of a labelled control RNA were fixed on a nylon membrane and subsequently developed with the anti-DIG-F_{ab}-hydroxylase conjugate as described in Example 8.

### Example 6

### Hybridization assay

RNA or DNA from samples immobilized on nylon membranes was hybridized with DIG-labelled RNA probes. Both with respect to RNA and with respect to DNA, the hybridization was carried out under identical conditions, except for the lower hybridization temperature in the case of the DNA-RNA hybridizations.

The membranes were first of all pre-hybridized in hybridizing buffer (50% formamide, 50 mM sodium phosphate pH 7.0, 7% SDS, 0.1% N-lauroylsarcosine, 5xSSC, 2% blocking reagent (Boehringer Mannheim) for 1 to 2 hours (65°C in the case of the RNA-RNA and 50°C in the case of the DNA-RNA hybridizations). The hybridization with the DIG-labelled probe was carried out for 16 to 90 hours and also at 65°C and 50°C, respectively. Immediately beforehand, the probe was heated to 98°C for 5 minutes and subsequently cooled on ice. After hybridization, the membrane was washed twice for 5 minutes in 2xSSC, 0.1% SDS (at ambient temperature) and twice for 15 minutes in 0.1xSSC, 0.1% SDS (at 65°C).

The detection of the hybridized probe was carried out applying the following procedure:
1. Washing in buffer 1 (150 mM NaCl, 100 mM maleic acid pH 7.5) for 2 minutes;
2. Saturating the membrane in buffer 2 (1% blocking reagent in buffer 1) for 30 to 60 minutes;
3. Incubating with anti-DIG-F_{ab}-hydroxylase conjugate (1:10,000 diluted in buffer 2; 75 mU/ml) for 30 minutes;
4. Washing twice in buffer 1 with 0.3% Tween-20 for 15 minutes;
5. Washing in buffer 3 (100 mM NaCl, 100 mM Tris/HCl pH 9.5) for 2 minutes.

The membrane, together with the substrate solution (CSPD [di-sodium-3-(4-methoxyspiro {1,2-dioxetane-3,2'-(5'-chloro)tricyclo[3.3.1.1^{3,7}]decan}-4-yl)phenylphosphate] diluted at a ratio of 1:100 in buffer 3), was welded into plastic foil and incubated for 30 minutes at 37°C. Thereafter, the substrate solution was pressed out from the foil, the foil was sealed by welding and the signals were represented by exposition on X-ray film. The optimum exposition times were between 30 minutes and 2 hours.

### Example 7

### Preparation of antisense DNA and hydroxylase-deficient CHO cells

Oligonucleotides and PLL conjugates are prepared according to J.P. Leonetti et al., Gene 72 (1988) 323-332.

β-anomeric oligodesoxynucleotide molecules (15-20mer) complementary to nucleotides of SEQ ID NO:1 were synthesized on a Biosearch Cyclone DNA synthesizer using a phosphoramidite method. As a control, random oligonucleotide molecules having the same length were prepared in identical fashion. The purity of oligonucleotide molecules was determined by electrophoresis through 15% polyacrylamide gels stained with etidium bromide.

### Preparation of an adenosine-derivatized support

To a solution of N⁶, 2'-(3')o-dibenzoyl-5'-dimethoxytrityl-adenosine, 2.15 g, 2.76 mmol (Kempe et al., Nucleic Acids Res. 10 (1982) 6695-6714) and dimethylamino-4-pyridine 0.506 g, 4.15 mmol in methylene chloride 12.3 ml were added succinic anhydride 0.415 mmol and triethylamine 0.58 ml, 4.15 mmol. The mixture was stirred for 2.5 h, poured onto 1 M aqueous triethylammonium hydrogen carbonate (120 ml), and the products were extracted with methylene chloride (3 x 150 ml). The combined organic layers were washed with water, dried over anhydrous sodium sulfate and evaporated to dryness. The residue was fractionated by silica gel chromatography using methanol-triethylamine-methylene chloride (0:1:99 to 2:1:97, v/v/v) as eluent. Fractions containing the pure 3'-o-hemisuccinate were combined and evaporated to dryness. The residue was dissolved in 1,2-dimethoxyethane and pentachlorophenol 0.783 g, 2.94 mmol and N,N'-dicyclohexylcarbodiimide 0.606 g, 2.94 mmol were added. The solution was stirred for 24 h and evaporated to dryness. The resulting residue was purified by silica gel chromatography using acetone-methylene chloride (5:95 to 8:92, v/v) as eluent. Fractions containing pure succinate diester were combined and evaporated to dryness. Precipitation of the residue from petroleum ether afforded a colorless powder (1.69 g) in 56% overall yield.

Long-chain alkylamine-controlled pore glass (5 g, from Sigma) was activated with triethylamine (0.3 ml, 1.5 mmol) in pyridine (10.5 ml). After evaporation of the solvent, the residue was suspended in a solution of succinate diester (1.69 g, 1.5 mmol) in dry pyridine (15 ml). After the mixture was stirred gently for 3 days at room temperature, the solid material was collected by suction, washed thoroughly with pyridine and methylene chloride, and dried. The glass beads were suspended in a capping solution (24 ml) made from acetic anhydride (0.186 ml, 1.98 mmol), 2,6-lutidine (0.198 ml, 1.71 mmol) and 4-dimethylaminopyridine (1.8 g, 14.7 mmol) in anhydrous tetrahydrofuran (30 ml). The mixture was stirred for 10 min and the glass beads were collected by suction, washed with tetrahydrofuran (2 x 20 ml) and methylene chloride (4 x 20 ml) and dried. Spectrophotometric measurement of the amount of dimethoxytrityl cation liberated by treating a portion of the functionalized adenosine-derivatized glass beads with 0.1 M toluene-sulfonic acid in acetonitrile indicated a loading of 24 µmol·g.

### Synthesis of oligos

(β)-Anomeric oligonucleotides were synthesized on a Biosearch Cyclone DNA synthesizer using the phosphoramidite method. The synthesis was initiated on an adenosine derivatized support prepared as described above.

### Conjugation of oligos to poly(L-lysine) (PLL)

The (β)-anomeric oligos (80 nmol) in 100 µm of 20 mM Na acetate (pH 4.4) were oxidized with 4.6 µmol Na metaperiodate for 30 min at 0°C in the dark. An equal volume of PLL (mean 14-kDa, Sigma) 80 nmol in 2 M NaCl, 0.2 M Na borate buffer (pH 8.4) and 100 µmol sodium cyanoborohydride were added. The mixture was incubated overnight at 20°C and then loaded on a Sephadex G-50 column equilibrated with 0.5 M NaCl, 20 mM Na acetate buffer (pH 6.0). Each fraction was assayed for its oligo-PLL content by absorbance at 260 nm and by the BCA protein assay (from Pierce). The conjugates were stored at -80°C.

### Transfection

Hydroxylase-positive CHO-wt cells were incubated with 0.9 µg oligo-PLL conjugate. Hydroxylase activity was measured according to W. Schlenzka et al. (1994) *(20)*. Cell extracts were added at 37°C to a mixture of 50 mM HEPES/NaOH (pH 7.4) at 37°C in the presence of 1 mM NADH, 0.5 mM FeSO₄, 10 µM CMP-[4,5,6,7,8,9-⁻¹⁴C]Neu5Ac and pig liver microsomes (∼20 µg protein in the test) solubilized in Triton X-100 (1.3%, by mass), the final volume of an assay being 30 µl. Solubilized microsomes were used as a source of cytochrome b₅ and cytochrome b₅ reductase L. Shaw et al. (1994) *(14)*. The assays, performed in duplicate, were stopped by the addition of 5 µl 1 M trichloroacetic acid. The precipitated material was removed by centrifugation and released [¹⁴C] sialic acids were analysed using radio TLC (L. Shaw and R. Schauer (1989) *(83)*.

Fractions from Q-Sepharose, Cibacron Blue 3GA-Agarose, Reactive Brown 10-Agarose and Hexyal Agarose chromatography were desalted on NAP-5 columns equilibrated with 50 mM HEPES/NaOH (pH 7.4) before measuring their hydroxylase activity.

### Example 8

### Production of Erythropoietin (EPO) in hydroxylase deficient CHO cells

Hydroxylase-deficient CHO cells are prepared by transfection of CHO wild-type cells with a replacement or insertion vector according to Figure 1.

DNA (20 µg) from the plasmid pPTFL13 described in Example 6 of EP-B 0 205 564 was digested with the restriction endonuclease Cla I to linearize the plasmid and was ligated to Cla I-digested DNA from the plasmid pAdD26SVp(A) 1 (2 µg) which contains an intact dihydrofolate reductase (DHFR) gene driven by an adenovirus major late promoter (Kaufman and Sharp (1982) *(21)*. This ligated DNA was used to transfect DHFR-negative CHO cells and following growth for two days, cells which incorporated at least one DHFR gene were selected in alpha media lacking nucleotides and supplemented with 10% dialyzed fetal bovine serum. Following growth for two weeks in selective media, colonies were removed from the original plates, pooled into groups of 10 to 100 colonies per pool, replated and grown to confluence in alpha media lacking nucleotides. The supernatant media from the pools grown prior to methotrexate selection were assayed for EPO by RIA. Pools which showed positive EPO production were grown in the presence of methotraxate (0.02 uM) and then subcloned and reassayed. A subclone was subjected to stepwise selection in increasing concentrations of MTX according to EP-B 0 205 564 (which is incorporated herein by reference). Such a subclone can be used for the production of EPO; EPO can be purified from cell supernatant by applying different standard chromatographic techniques as described, for instance, in EP-B 0 209 539 *(64)*.

### Example 9

### Carrying out the determination of the sialic acid

Determination of the sialic acid is carried out by enzymatically splitting the sialic acid and subsequently performing chromatographic determination of the sialic acid by means of "Dionex-HPEAC".

To an EPO sample solution in sodium phosphate buffer, pH 7.2 (about 90 to 180 µl)) were added 5 µl neuramidase from Arthrobacter ureafaciens 1 U/100 µl Boehringer Mannheim, No: 269 611) in sodium phosphate buffer to a final concentration of 2 nmol EPO protein in 200 µl. Mixing is carried out, and incubation is performed for 7 hours at 37°C. 100 µl of sample volume is withdrawn and mixed with 300 µl water. This diluted sample solution is used to carry out the chromatographic determination of sialic acid by means of "Dionex-HPAEC". Detection is carried out with an amperometric detector (PAD; Dionex Corp., Sunnyvale, USA) in line with the HPEAC. Separation of Neu5Ac and Neu5Gc is achieved with a linear gradient using 100 mM NaOH (eluent A) and 500 mM sodium acetate (eluent B). Eluent B is increased from 0% to 100% in 40 minutes. NeuAc elutes before Neu5Gc does under these conditions and both sialic acids can be quantified using Neu5Ac and Neu5Gc as standards (concentration: 1 to 10 nmol for Neu5Ac and 0.05 to 0.5 nmol for Neu5Gc). The PAD setting for the detection of Neu5Ac 8s 3000 nA and for Neu5Gc, 100 nA.

### References

- *(1)*: Schauer, R., Trends Biochem. Sci. 10 (1985) 357-360
- *(2)*: Varki, A., Glycobiology 2 (1992a) 25-40
- *(3)*: Varki, A., Curr. Opin. Cell Biol. 4 (1992b) 257-266
- *(4)*: Imai, N., et al., Eur. J. Biochem. 194 (1990) 457-462
- *(5)*: Corfield, A.P., and Schauer, R., Sialic Acids, Cell Biol. Monogr. 10, Schauer, R. (ed.), Springer, Vienna (1982) 5-55
- *(6)*: Reuter, G., et al., Proceedings of the Japanese-German Symposium on Sialic Acids, Schauer, R., and Yamakawa, T., (eds.), Kieler Verlag Wissenschaft und Bildung, Kiel (1988) 8-89
- *(7)*: Bouhours, D., and Bouhours, J.-F., J. Biol. Chem. 258 (1983) 299-304
- *(8)*: Deicher, H., I. Mitteilung. Z. Hyg. 106 (1926) 561-579
- *(9)*: Higashi, H., et al. Cancer Res. 45 (1985) 3796-3802
- *(10)*: Higashi, H., Trends in Glycosci. and Glycotechnol. 2 (1990) 7-15
- *(11)*: Higashihara, T., et al., Int. Arch, Allergy Appl. Immunol. 95 (1991) 231-235
- *(12)*: Shaw, L., and Schauer, R., Biol. Chem. Hoppe-Seyler 369 (1988) 477-486
- *(13)*: Kozutsumi, Y., et al., J. Biochem 108 (1990) 704-706
- *(14)*: Shaw, L., et al., Eur. J. Biochem. 219 (1994) 1001-1011
- *(15)*: Lepers, A., et al., FEBS Lett. 250 (1989) 245-250
- *(16)*: Higa, H.-H., and Paulson, J.C., J. Biol. Chem. 260 (1985) 8838-8849
- *(17)*: Hokke, C.H., et al., FEBS Lett. 275 (1990) 9-14
- *(18)*: Kawano, T., et al., J. Biol. Chem. 269 (1994) 9024-9029
- *(19)*: Schneckenburger, P., et al., Glycoconjugate J. 11 (1994) 194-203
- *(20)*: Schlenzka, W., et al., Glycobiology 4 (1994) 675-683
- *(21)*: Kaufman and Sharp, Mol. and Cell Biol. 2 (1982) 1304-1319
- *(22)*: Sambrook et al., "Expression of cloned genes in E. coli" in Molecular Cloning: A laboratory manual (1989), Cold Spring Harbor Laboratory Press, New York, USA
- *(23)*: B.D. Hames, S.G. Higgins, Nucleic acid hybridisation - a practical approach (1985) IRL Press, Oxford, England
- *(24)*: Höltke and Kessler "The DIG System User's Guide for Filter Hybridization (1990), Boehringer Mannheim GmbH, Germany
- *(25)*: Büttner et al., Mol. Cell. Biol. 11 (1991) 3573 - 3583
- *(26)*: Ausubel I., Frederick M., Current Protocols in Mol. Biol. (1992), John Wiley and Sons, New York
- *(27)*: WO 89/06698
- *(28)*: EP-A 0 200 362
- *(29)*: USP 2,915,082
- *(30)*: EP-A 0 063 879
- *(31)*: EP-A 0 173 251
- *(32)*: EP-A 0 128 018
- *(33)*: J. Mol. Biol. 98 (1975) 503
- *(34)*: Proc. Natl. Acad. Sci. USA 76 (1979), 3683
- *(35)*: M. Cooney et al., Science 241 (1988) 456
- *(36)*: Duvall-Valentine et al., PNAS 89 (1992) 504-508
- *(37)*: J.-J.Toulmé and C. Hélène, Gene 72 (1988) 51-58
- *(38)*: S. Beaucage and M. Caruthers, Tetrahedon Letters 37 (1981) 3557
- *(39)*: G. Zon and T. Geiser, Anticancer Drug Des. 6 (1991) 539
- *(40)*: C.A. Stein et al., Pharmacol. Ter. 52 (1991) 365
- *(41)*: P. Miller, Biotechnology 9 (1991) 358
- *(42)*: P.S. Miller et al., Nucleosides and Nucleotides 6 (1985) 769-776
- *(43)*: M. Lemaitre et al., PNAS USA 84 (1987) 648-652
- *(44)*: WO 92/20316
- *(45)*: USP 5,166,320
- *(46)*: WO 92/19281
- *(47)*: WO 92/13570
- *(48)*: EP-A 0 388 758
- *(49)*: WO 93/07283
- *(50)*: WO 92/17210
- *(51)*: WO 91/17773
- *(52)*: WO 93/04701
- *(53)*: PCT/EP94/01147
- *(54)*: WO 91/06309
- *(55)*: WO 91/17424
- *(56)*: Wu and Wu, J. Biol. Chem. (1987) 4429-4432
- *(57)*: Wu et al., J. Biol. Chem. 263 (1988) 14621-14624
- *(58)*: P. Midoux et al., Nucleic Acid Res. 21 (1993) 871-878
- *(59)*: Plank et al., Bioconjugate Chem. 3 (1992) 533-539
- *(60)*: Cotten et al., PNAS USA 89 (1992) 6094-6098
- *(61)*: Wagner et al., PNAS USA 89 (1992) 6099-6103
- *(62)*: Wagner et al., PNAS USA 89 (1992) 7934-7938
- *(63)*: G. Jung et al., Biochem. Biophys. Rest Commun. 101 (1981) 599-606
- *(64)*: EP-B 0 209 539
- *(65)*: W.F. Kern et al., Leukemia and Lymphoma 12 (1993) 1-10
- *(66)*: Brinckmann et al., Gene 85 (1989) 109-114
- *(67)*: Earhart et al., FEMS Microbiology Letters 6 (1979) 277-280
- *(68)*: Grodberg and Dunn, J. Bacteriol. 170 (1988) 1245-1253
- *(69)*: EP 0 241 022
- *(70)*: EP 0 364 926
- *(71)*: EP 0 219 874
- *(72)*: DE-A 40 37 196
- *(73)*: Umesono et al., Cell 65 (1991) 1255-1266
- *(74)*: Methods of Enzymology 185 (Gene Expression Technology), ed. David V. Goeddel, Academic Press 1991, section
- *(75)*: US Patent No. 5,272,071
- *(76)*: M.R. Capecchi, Science 244 (1989) 1288-1292
- *(77)*: G.Y. Wu et al., J. Biol. Chem. 266 (1991) 14338-14342
- *(78)*: WO 91/13151
- *(79)*: USP 4,399,216
- *(80)*: USP 4,634,665
- *(81)*: A.L. Joyner, Gene Targeting, IRL Press (1993)
- *(82)*: R. Schauer et al., Advances in Carbohydrate Chemistry and Biochemistry 40 (1982) 131-234
- *(83)*: L. Shaw and R. Schauer, Biochem. J. 263 (1989) 355-363

## Claims

1. An isolated protein substantially free of contamination with cytochrome b₅ and cytochrome b₅ reductase, which
a) is a product of a prokaryotic or eukaryotic expression of an exogenous DNA,
b) catalyzes the conversion of CMP-Neu5A to CMP-Neu5Gc, and
c) is coded by the DNA sequence shown in SEQ ID NO:1;
d) is coded by DNA sequences, which hybridize with SEQ ID NO:1 under stringent conditions; or
e) is coded by DNA sequences, which, if there was no degeneracy of the genetic code, would hybridize under stringent conditions with the sequences defined in c) to d).

2. The protein according to claim 1, said protein being coded by a sequence, which hybridizes under stringent conditions with the nucleotides 42 to 1307 of SEQ ID NO:1.

3. An isolated nucleic acid molecule, which secures expression in a prokaryotic or eukaryotic host cell of a protein with CMP-N-acetylneuranic acid hydroxylase activity and is selected from the group of
a) DNA molecules shown in SEQ ID NO:1 or the complementary sequences;
b) nucleic acid molecules, which hybridize with SEQ ID NO:1 under stringent conditions; or
c) nucleic acid molecules, which, if there was no degeneracy of the genetic code, would hybridize under stringent conditions with one of the sequences stated in a) or b).

4. The nucleic acid molecule according to claim 3, said nucleic acid molecule hybridizing under stringent conditions with the nucleotides 42 to 1307 of SEQ ID NO:1.

5. A method for the detection of nucleic acid molecules, which code for a protein having CMP-N-acetylneuramic acid hydroxylase activity, which is characterized in that the sample to be examined is incubated with a nucleic acid probe, which is selected from the group comprising nucleic acid molecules, which hybridize under stringent conditions with nucleotides 42 to 1307 of SEQ ID NO:1, the nucleic acid probe is incubated with the nucleic acid molecule from the sample and the hybridization of the nucleic acid molecule in the sample and nucleic acid probe is detected, if desired, via a further binding partner.

6. An oligonucleotide molecule, which hybridizes under stringent conditions in a manner which is specific for nucleic acid molecule with SEQ ID NO:1 or related RNA sequences, which code for a protein with CMP-N-acetylneuramic acid hydroxylase activity under extracellular or intracellular conditions.

7. The oligonucleotide molecule according to claim 6, which inhibits the expression of CMP-N-acetylneuramic acid hydroxylase.

8. The oligonucleotide molecule according to claim 6 or 7, characterized in binding to CMP-N-acetylneuramic acid hydroxylase mRNA.

9. The oligonucleotide molecule according to claims 6 to 8, characterized in that as oligonucleotide molecules, oligonucleotide molecule derivatives such as phosphotriester, methylphosphonates or phosphothioates or substituted oligonucleotides such as acridine-substituted or interchelating coupled oligonucleotide molecules or α-anomers are used.

10. The oligonucleotide molecule according to claims 6 to 9, having 15 to 50 bases.

11. A soluble complex for targeting to mammalian cells an oligonucleotide molecule, which hybridizes in a manner which is specific for a nucleic acid molecule, which codes for a protein with CMP-N-acetylneuramic acid hydroxylase activity with a part or all of SEQ ID NO:1 or related RNA sequences complexed with an oligonucleotide molecule binding agent, which complexes the oligonucleotide molecule under extracellular conditions and releases said oligonucleotide under intracellular conditions as an oligonucleotide molecule specifically bindable to said nucleic acid molecule.

12. The soluble complex according to claim 11, wherein the oligonucleotide molecule binding agent is a polycation, such as polylysine.

13. The soluble complex according to claim 11 or 12, wherein the complex contains in addition a cell specific binding agent.

14. A method for the production of a second mammalian cell in vitro, which is CMP-N acetyl neuraminic acid hydroxylase activity-deficient, by transfecting a first mammalian cell, which contains said hydroxylase activity, with a nucleic acid molecule, which hybridizes under stringent conditions with the genome of said second cell, in a manner which is specific for the gene region of said hydroxylase activity coding gene and thereby modifies the genome of said first cell in such a manner that said first cell does not further contain hydroxylase activity, isolating from the cell mixture at least one second cell, which is deficient in said hydroxylase activity.

15. The use of a mammalian cell, which contains in its initial state a CMP-N acetyl neuraminic acid hydroxylase activity and subsequently is modified in such a manner that said cell no longer contains hydroxylase activity, for the production of a recombinant glycoprotein, wherein said cell is transfected with a nucleic acid molecule, which secures the expression of said recombinant protein, culturing said cells and isolating said recombinant glycoprotein from the cells or the supernatant.

16. Glycoconjugates, especially glycoproteins obtained from cells according to claim 15, which are characterized by the absence of Neu5Gc.

17. Erythropoietin obtained from cells, especially CHO cells according to claim 15, which is characterized by the absence of Neu5Gc.


SEQUENCE LISTING

(1) GENERAL INFORMATION:

(i) APPLICANT:
(A) NAME: BOEHRINGER MANNHEIM GMBH
(B) STREET: Sandhofer Str. 116
(C) CITY: Mannheim
(E) COUNTRY: Germany
(F) POSTAL CODE (ZIP): D-68305
(G) TELEPHONE: 0885660-3446
(H) TELEFAX: 0885660-3451

(ii) TITLE OF INVENTION: Nucleic acid coding for
CMP-N-acetylneuraminic acid hydroxylase and its use for
the production of modified glycoproteins

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30B (EPO)

(2) INFORMATION FOR SEQ ID NO: 1:

(i) SEQUENCE CHARACTERISTICS:
(A) LENGTH: 1986 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION:42..1307

(xi) SEQUENCE DESCRIPTION: SEQ ID NO: 1:

TTACTCACGC CTGCATGGAC CTCAAGCTGG GGGACAAGAG A ATG GTG TTC GAC 53
Met Val Phe Asp
1

CCT TGG TTA ATC GGT CCT GCT TTT GCG CGA GGA TGG TGG TTA CTA CAC 101
Pro Trp Leu Ile Gly Pro Ala Phe Ala Arg Gly Trp Trp Leu Leu His
5                   10                  15                  20

GAG CCT CCA TCT GAT TGG CTG GAG AGG CTG AGC CGC GCA GAC TTA ATT 149
Glu Pro Pro Ser Asp Trp Leu Glu Arg Leu Ser Arg Ala Asp Leu Ile
                25                  30                  35

TAC ATC AGT CAC ATG CAC TCA GAC CAC CTG AGT TAC CCA ACA CTG AAG 197
Tyr Ile Ser His Met His Ser Asp His Leu Ser Tyr Pro Thr Leu Lys
            40                  45                  50

AAG CTT GCT GAG AGA AGA CCA GAT GTT CCC ATT TAT GTT GGC AAC ACG 245
Lys Leu Ala Glu Arg Arg Pro Asp Val Pro Ile Tyr Val Gly Asn Thr
        55                  60                  65

GAA AGA CCT GTA TTT TGG AAT CTG AAT CAG AGT GGC GTC CAG TTG ACT 293
Glu Arg Pro Val Phe Trp Asn Leu Asn Gln Ser Gly Val Gln Leu Thr
    70                  75                  80

AAT ATC AAT GTA GTG CCA TTT GGA ATA TGG CAG CAG GTA GAC AAA AAT 341
Asn Ile Asn Val Val Pro Phe Gly Ile Trp Gln Gln Val Asp Lys Asn
85                  90                  95                  100

CTT CGA TTC ATG ATC TTG ATG GAT GGC GTT CAT CCT GAG ATG GAC ACT 389
Leu Arg Phe Met Ile Leu Met Asp Gly Val His Pro Glu Met Asp Thr
                105                 110                 115

TGC ATT ATT GTG GAA TAC AAA GGT CAT AAA ATA CTC AAT ACA GTG GAT 437
Cys Ile Ile Val Glu Tyr Lys Gly His Lys Ile Leu Asn Thr Val Asp
            120                 125                 130

TGC ACC AGA CCC AAT GGA GGA AGG CTG CCT ATG AAG GTT GCA TTA ATG 485
Cys Thr Arg Pro Asn Gly Gly Arg Leu Pro Met Lys Val Ala Leu Met
        135                 140                 145

ATG AGT GAT TTT GCT GGA GGA GCT TCA GGC TTT CCA ATG ACT TTC AGT 533
Met Ser Asp Phe Ala Gly Gly Ala Ser Gly Phe Pro Met Thr Phe Ser
    150                 155                 160

GGT GGA AAA TTT ACT GAG GAA TGG AAA GCC CAA TTC ATT AAA ACA GAA 581
Gly Gly Lys Phe Thr Glu Glu Trp Lys Ala Gln Phe Ile Lys Thr Glu
165                 170                 175                 180

AGG AAG AAA CTC CTG AAC TAC AAG GCT CGG CTG GTG AAG GAC CTA CAA 629
Arg Lys Lys Leu Leu Asn Tyr Lys Ala Arg Leu Val Lys Asp Leu Gln
                185                 190                 195

CCC AGA ATT TAC TGC CCC TTT CCT GGG TAT TTC GTG GAA TCC CAC CCA 677
Pro Arg Ile Tyr Cys Pro Phe Pro Gly Tyr Phe Val Glu Ser His Pro
            200                 205                 210

GCA GAC AAG TAT ATT AAG GAA ACA AAC ATC AAA AAT GAC CCA AAT GAA 725
Ala Asp Lys Tyr Ile Lys Glu Thr Asn Ile Lys Asn Asp Pro Asn Glu
        215                 220                 225

CTC AAC AAT CTT ATC AAG AAG AAT TCT GAG GTG GTA ACC TGG ACC CCA 773
Leu Asn Asn Leu Ile Lys Lys Asn Ser Glu Val Val Thr Trp Thr Pro
    230                 235                 240

AGA CCT GGA GCC ACT CTT GAT CTG GGT AGG ATG CTA AAG GAC CCA ACA 821
Arg Pro Gly Ala Thr Leu Asp Leu Gly Arg Met Leu Lys Asp Pro Thr
245                 250                 255                 260

GAC AGC AAG GGC ATC GTA GAG CCT CCA GAA GGG ACT AAG ATT TAC AAG 869
Asp Ser Lys Gly Ile Val Glu Pro Pro Glu Gly Thr Lys Ile Tyr Lys
                265                 270                 275

GAT TCC TGG GAT TTT GGC CCA TAT TTG AAT ATC TTG AAT GCT GCT ATA 917
Asp Ser Trp Asp Phe Gly Pro Tyr Leu Asn Ile Leu Asn Ala Ala Ile
            280                 285                 290

GGA GAT GAA ATA TTT CGT CAC TCA TCC TGG ATA AAA GAA TAC TTC ACT 965
Gly Asp Glu Ile Phe Arg His Ser Ser Trp Ile Lys Glu Tyr Phe Thr
        295                 300                 305

TGG GCT GGA TTT AAG GAT TAT AAC CTG GTG GTC AGG ATG ATT GAG ACA 1013
Trp Ala Gly Phe Lys Asp Tyr Asn Leu Val Val Arg Met Ile Glu Thr
    310                 315                 320

GAT GAG GAC TTC AGC CCT TTG CCT GGA GGA TAT GAC TAT TTG GTT GAC 1061
Asp Glu Asp Phe Ser Pro Leu Pro Gly Gly Tyr Asp Tyr Leu Val Asp
325                 330                 335                 340

TTT CTG GAT TTA TCC TTT CCA AAA GAA AGA CCA AGC CGG GAA CAT CCA 1109
Phe Leu Asp Leu Ser Phe Pro Lys Glu Arg Pro Ser Arg Glu His Pro
                345                 350                 355

TAT GAG GAA ATT CGG AGC CGG GTT GAT GTC ATC AGA CAC GTG GTA AAG 1157
Tyr Glu Glu Ile Arg Ser Arg Val Asp Val Ile Arg His Val Val Lys
            360                 365                 370

AAT GGT CTG CTC TGG GAT GAC TTG TAC ATA GGA TTC CAA ACC CGG CTT 1205
Asn Gly Leu Leu Trp Asp Asp Leu Tyr Ile Gly Phe Gln Thr Arg Leu
        375                 380                 385

CAG CGG GAT CCT GAT ATA TAC CAT CAT CTG TTT TGG AAT CAT TTT CAA 1253
Gln Arg Asp Pro Asp Ile Tyr His His Leu Phe Trp Asn His Phe Gln
    390                 395                 400

ATA AAA CTC CCC CTC ACA CCA CCT GAC TGG AAG TCC TTC CTG ATG TGC 1301
Ile Lys Leu Pro Leu Thr Pro Pro Asp Trp Lys Ser Phe Leu Met Cys
405                 410                 415                 420

TCT GGG TAGAGAGGAC CTGAGCTGTC CCAGGGGTGC CCAACAACAT GAAAAAATCA 1357
Ser Gly

AGAATTTATT GCTGCTACGT CAAAGCTTAT ACCAGAGATT ATGCCTTATA GACATTAGCA 1417

ATGGATAATT ATATGTTGCA CTTGTGAAAT GTGCACATAT CCTGTTTATG AATCACCACA 1477

TAGCCAGATT ATCAATATTT TACTTATTTC GTAAAAAATC CACAATTTTC CATAACAGAA 1537

TCAACGTGTG CAATAGGAAC AAGATTGCTA TGGAAAACGA GGGTAACAGG AGGAGATATT 1597

AATCCAAGCA TAGAAGAAAT AGACAAATGA GGGGCCATAA GGGGAATATA GGGAAGAGAA 1657

AAAAATTAAG ATGGAATTTT AAAAGGAGAA TGTAAAAAAT AGATATTTGT TCCTTAATAG 1717

GTTGATTCCT CAAATAGAGC CCATGAATAT AATCAAATAG GAAGGGTTCA TGACTGTTTT 1777

CAATTTTTCA AAAAGCTTTG TTGAAATCAT AGACTTGCAA AACAAGGCTG TAGAGGCCAC 1837

CCTAAAATGG AAAATTTCAC TGGGACTGAA ATTATTTTGA TTCAATGACA AAATTTGTTA 1897

TTTACTGCGG ATTATAAACT CTAACAAATA GCGATCTCTT TGCTTCATAA AAACATAAAC 1957

ACTAGCTAGT AATAAAATGA GTTCTGCAG 1986

(2) INFORMATION FOR SEQ ID NO: 2:

(i) SEQUENCE CHARACTERISTICS:
(A) LENGTH: 422 amino acids
(B) TYPE: amino acid
(D) TOPOLOGY: linear

(ii) MOLECULE TYPE: protein
(xi) SEQUENCE DESCRIPTION: SEQ ID NO: 2:

Met Val Phe Asp Pro Trp Leu Ile Gly Pro Ala Phe Ala Arg Gly Trp
1               5                   10                  15

Trp Leu Leu His Glu Pro Pro Ser Asp Trp Leu Glu Arg Leu Ser Arg
            20                  25                  30

Ala Asp Leu Ile Tyr Ile Ser His Met His Ser Asp His Leu Ser Tyr
        35                  40                  45

Pro Thr Leu Lys Lys Leu Ala Glu Arg Arg Pro Asp Val Pro Ile Tyr
    50                  55                  60

Val Gly Asn Thr Glu Arg Pro Val Phe Trp Asn Leu Asn Gln Ser Gly
65                  70                  75                  80

Val Gln Leu Thr Asn Ile Asn Val Val Pro Phe Gly Ile Trp Gln Gln
                85                  90                  95

Val Asp Lys Asn Leu Arg Phe Met Ile Leu Met Asp Gly Val His Pro
            100                 105                 110

Glu Met Asp Thr Cys Ile Ile Val Glu Tyr Lys Gly His Lys Ile Leu
        115                 120                 125

Asn Thr Val Asp Cys Thr Arg Pro Asn Gly Gly Arg Leu Pro Met Lys
    130                 135                 140

Val Ala Leu Met Met Ser Asp Phe Ala Gly Gly Ala Ser Gly Phe Pro
145                 150                 155                 160

Met Thr Phe Ser Gly Gly Lys Phe Thr Glu Glu Trp Lys Ala Gln Phe
                165                 170                 175

Ile Lys Thr Glu Arg Lys Lys Leu Leu Asn Tyr Lys Ala Arg Leu Val
            180                 185                 190

Lys Asp Leu Gln Pro Arg Ile Tyr Cys Pro Phe Pro Gly Tyr Phe Val
        195                 200                 205

Glu Ser His Pro Ala Asp Lys Tyr Ile Lys Glu Thr Asn Ile Lys Asn
    210                 215                 220

Asp Pro Asn Glu Leu Asn Asn Leu Ile Lys Lys Asn Ser Glu Val Val
225                 230                 235                 240

Thr Trp Thr Pro Arg Pro Gly Ala Thr Leu Asp Leu Gly Arg Met Leu
                245                 250                 255

Lys Asp Pro Thr Asp Ser Lys Gly Ile Val Glu Pro Pro Glu Gly Thr
            260                 265                 270

Lys Ile Tyr Lys Asp Ser Trp Asp Phe Gly Pro Tyr Leu Asn Ile Leu
        275                 280                 285

Asn Ala Ala Ile Gly Asp Glu Ile Phe Arg His Ser Ser Trp Ile Lys
    290                 295                 300

Glu Tyr Phe Thr Trp Ala Gly Phe Lys Asp Tyr Asn Leu Val Val Arg
305                 310                 315                 320

Met Ile Glu Thr Asp Glu Asp Phe Ser Pro Leu Pro Gly Gly Tyr Asp
                325                 330                 335

Tyr Leu Val Asp Phe Leu Asp Leu Ser Phe Pro Lys Glu Arg Pro Ser
            340                 345                 350

Arg Glu His Pro Tyr Glu Glu Ile Arg Ser Arg Val Asp Val Ile Arg
        355                 360                 365

His Val Val Lys Asn Gly Leu Leu Trp Asp Asp Leu Tyr Ile Gly Phe
    370                 375                 380

Gln Thr Arg Leu Gln Arg Asp Pro Asp Ile Tyr His His Leu Phe Trp
385                 390                 395                 400

Asn His Phe Gln Ile Lys Leu Pro Leu Thr Pro Pro Asp Trp Lys Ser
                405                 410                 415

Phe Leu Met Cys Ser Gly
            420